(19) Europäisches Patentamt
European Patent Office
Office européen des brevets

(11) Numéro de publication: **0 322 279 B1**

(12) # FASCICULE DE BREVET EUROPEEN

(45) Date de publication de fascicule du brevet: **14.04.93**

(51) Int. Cl.5: **C07C 231/00**, C07C 233/15, C07C 233/56

(21) Numéro de dépôt: **88403172.5**

(22) Date de dépôt: **14.12.88**

(54) **Procédé d'acylation d'un N,N-diallylaniline.**

(30) Priorité: **23.12.87 FR 8718012**

(43) Date de publication de la demande:
**28.06.89 Bulletin 89/26**

(45) Mention de la délivrance du brevet:
**14.04.93 Bulletin 93/15**

(84) Etats contractants désignés:
**AT BE CH DE ES FR GB GR IT LI LU NL SE**

(56) Documents cités:
**DE-A- 2 334 632**

(73) Titulaire: **RHONE POULENC CHIMIE**
**25 Ouai Paul Doumer**
**F-92408 Courbevoie Cédex(FR)**

(72) Inventeur: **Denis, Jean-Pierre**
**Route des Terres Fortes Chamblet**
**F-03170 Doyet(FR)**
Inventeur: **Desmurs, Jean-Roger**
**La Jonquière Route De Ternay**
**F-69360 Communay(FR)**
Inventeur: **Lecouve, Jean-Pierre**
**23E rue de l'Oratoire**
**F-69300 Caluire(FR)**

(74) Mandataire: **Le Pennec, Magali**
**RHONE-POULENC INTERSERVICES Service**
**Brevets Chimie 25, Ouai Paul Doumer**
**F-92408 Courbevoie Cédex (FR)**

EP 0 322 279 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé d'acylation d'une N,N-diallylaniline. Elle concerne plus particulièrement l'acylation de la N,N-diallylmétatrifluorométhylaniline.

Selon le brevet FR 2 305 434 il est connu que la N-dichloracétyl N-allylmétatrifluorométhylaniline est un composé servant de matière première pour la préparation d'un herbicide ayant une forte potentialité, la N-métatrifluorométhylphényl-3 chloro-3 chlorométhyl-4 pyrrolidone-2 de formule générale

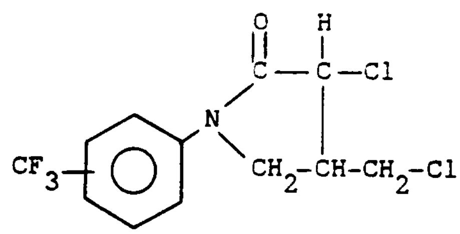

Le procédé de préparation de cet herbicide consiste à cycliser la N-dichloracétyl N-allylmétatrifluorométhylaniline selon la réaction suivante

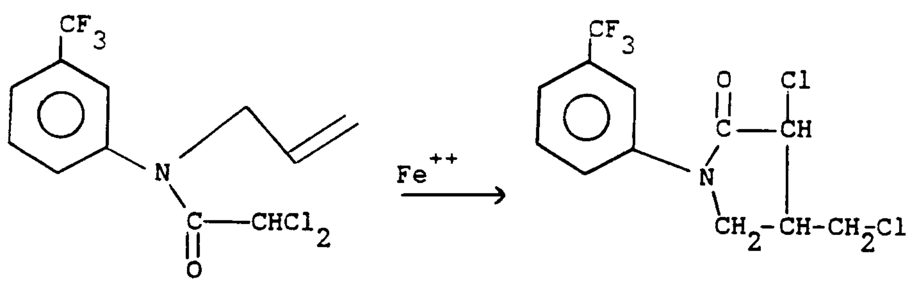


Le problème d'obtention de cet herbicide réside dans la préparation du dérivé N-allylique et N-acylé. En effet lorsque l'on commence par procéder à l'allylation de l'aniline celle-ci conduit à une quantité non négligeable de dérivés diallyliques qui doivent être éliminés afin de disposer du produit monoallylé qui est ensuite acylé. Lorsque l'on veut procéder à une monoallylation on doit suivre les étapes du procédé décrit dans le brevet précédemment cité.

Ainsi dans ce brevet FR 2 305 434 le prodédé de préparation de la N-allyl N-dichloracétyl aniline est réalisé selon les 4 étapes suivantes

- dans une première étape on protège un atome d'hydrogène par l'anhydride acétique,
- dans une deuxième étape on réalise l'allylation,
- dans une troisième étape on élimine le groupe protecteur acylé,
- dans une quatrième étape on procède à l'acylation par le chlorure de diacétyle selon le schéma suivant :
  . première étape

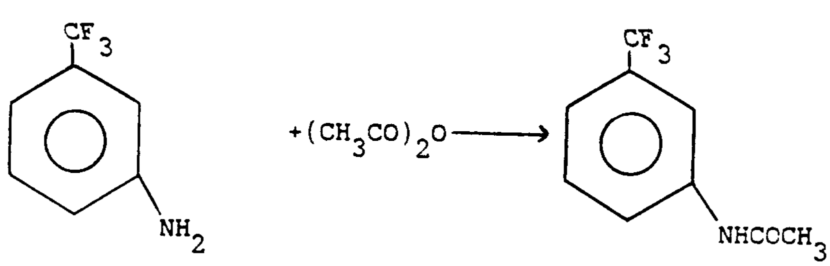

. deuxième étape

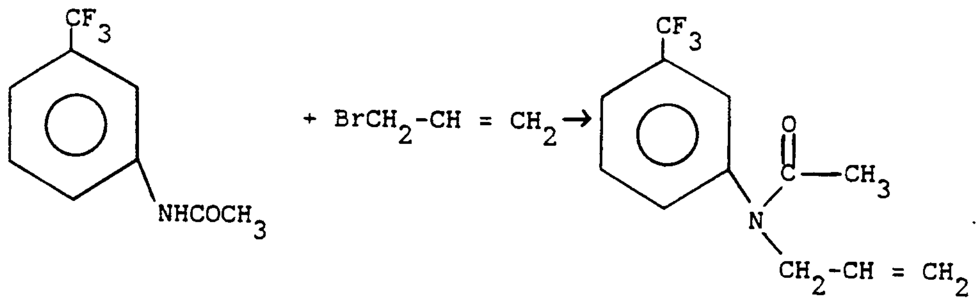


. troisième étape

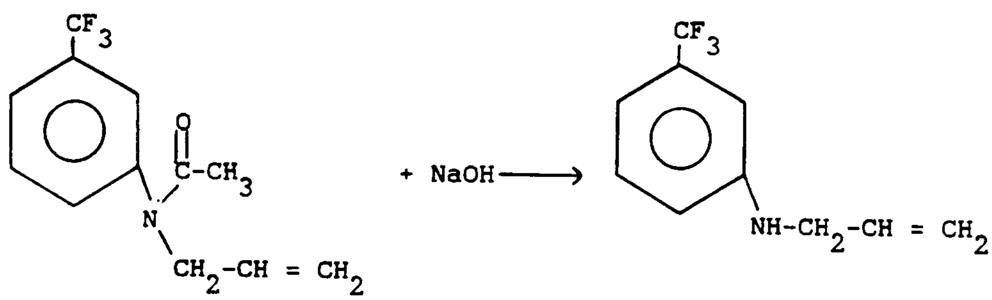


. quatrième étape

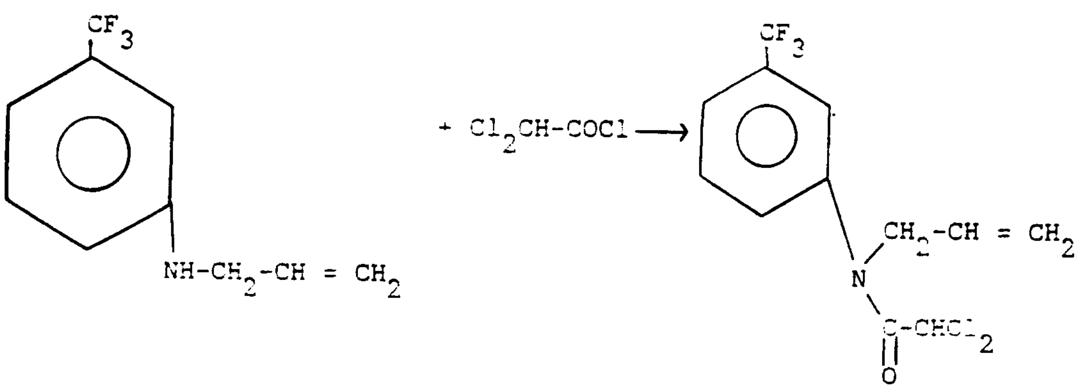


Le rendement obtenu après une telle succession d'étapes annoncé dans le brevet FR 2 305 434 exemple 7 est de l'ordre de 36 % ce qui est très faible et augmente d'autant plus le coût de production de l'herbicide final.

La présente invention a permis de rentabiliser ce procédé en évitant de passer par les protections intermédiaires qu'exigeait un tel procédé. En effet il est apparu par la présente invention qu'il était possible de passer directement d'une N,N-diallylaniline à une N-allyl N-acylaniline sans isoler l'intermédiaire monoallylé.

La présente invention a donc pour objet un procédé de préparation de N-allyl N-acylaniline caractérisé en ce qu'on met en contact une N,N-diallylaniline ou un mélange de N-mono et N,N-diallyl anilines avec un halogénure d'acide carboxylique de préférence en présence d'un catalyseur métallique choisi parmi le palladium et le cuivre avec la condition que lorsque le catalyseur est le palladium, on n'utilise pas les phosphines pour en potentialiser l'action.

Dans le document DE-A-2 334 632 qui a pour objet la désallylation au moyen d'anhydride carboxylique, il est indiqué que le palladium pris sans les phosphines ne marchent pas pour catalyser la désallylation.

La présente invention peut être appliquée sur un mélange de N-allyl et de N,N-diallyl anilines aussi bien que sur une solution pure de N,N-diallylaniline.

Il est connu selon l'article de P. CAUBERE et J.C. MADELMONT (C.R. Academie des sciences, 1972, 275, 1306) de couper des amines tertiaires aliphatiques ayant une chaîne insaturée sur l'azote par des chlorures d'acides en présence de cuivre dans le tétrahydrofuranne. Les halogénures cuivreux permettent seuls et non les sels cuivriques la coupure de la liaison azote-carbone, ils sont d'autre part utilisés en quantité stoechiométrique par rapport à l'amine tertiaire à couper.

En outre la basicité des amines de la présente invention est très faible comparée à celle des amines tertiaires aliphatiques, il n'était donc pas évident que l'amine puisse réagir avec le chlorure d'acide pour former l'acylium intermédiaire qui se décompose ensuite en amide.

Il est précisé dans cet article que la réaction est d'autant plus difficile que l'encombrement des substituants portés par l'atome d'azote et le groupe acylé est important. Dans la présente invention la taille des substituants benzénique et dichlorométhyle est importante.

Dans le cadre de la présente invention on peut utiliser un catalyseur métallique à base de palladium ou de cuivre. Ces catalyseurs peuvent se présenter sous forme de sels, d'oxydes ou de complexes.

Parmi les sels, oxydes et complexes des métaux précédemment cités, à l'état d'oxydation II, on peut mentionner : les chlorures de palladium, de cuivre, l'acétate de palladium, le chlorure de palladium complexé par le benzonitrile. Parmi les complexes des métaux à l'état d'oxydation zéro on peut citer le palladium dibenzylidèneacétone, et parmi ceux présentant le degré d'oxydation I on peut citer le chlorure cuivreux.

Parmi les N,N-diallylanilines utilisables dans le cadre de la présente invention on peut citer les composés de formule générale (I)

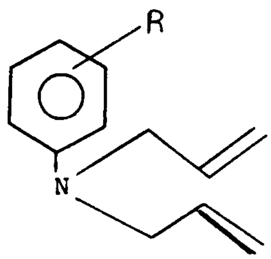

(I)

dans lesquels R représente un groupe halogéné, alkyle ayant 1 à 6 atomes de carbone, alcoxy ayant 1 à 6 atomes de carbone, alkylthio ayant 1 à 6 atomes de carbone, halogénoalkyl, halogénoalcoxy, halogénoalkylthio ayant 1 à 6 atomes de carbone, nitro, cyano.

On préfère tout particulièrement utiliser la N,N-diallylmétatrifluorométhylaniline.

Parmi les halogénures d'acides on peut utiliser les chlorures ou bromures d'acides carboxyliques ayant 2 à 12 atomes de carbone. Ces acides peuvent être éventuellement halogénés ainsi dans le cadre de la présente invention on préfère utiliser le chlorure de dichloroacétyle.

La réaction peut avoir lieu dans tout solvant tel que notamment :

- l'acétonitrile
- le benzene et les solvants aromatiques
- les alkanes ayant au moins 6 atomes de carbone
- l'eau
- le benzonitrile
- le diméthylformamide
- la N méthylpyrrolidone
- le nitrobenzène
- l'halogénure d'acide utilise dans la réaction.

Pour une meilleure mise en oeuvre de l'invention on préfère utiliser une quantité de catalyseur telle que le rapport molaire du catalyseur métallique à la N,N-diallylaniline est compris entre 0,1 et 5 % et de préférence d'environ 2,5 %. Le rapport molaire de l'halogénure d'acide à l'allylaniline est de préférence supérieur à 1.

La quantité de solvant utilisée est telle que la concentration de la N-allylaniline et/ou de la N,N-diallylaniline dans le solvant est de préférence supérieure à 50 g/litre.

Les conditions réactionnelles avantageuses pour la mise en oeuvre d'une telle réaction sont les suivantes. La température est de préférence comprise entre 20 °C et 120 °C. La pression utilisée est

avantageusement la pression atmosphérique bien qu'une pression supérieure ne soit en aucun cas nuisible.

L'ensemble de l'invention sera plus complètement décrite à l'aide des exemples suivants qui ne doivent en aucun cas être considérés comme limitatifs.

Dans les exemples suivants on entend par :

TT = le taux de transformation du produit de départ

$$TT = \frac{\text{nombre de moles de produit transformé}}{\text{nombre de moles de produit initial}}$$

RR = le rendement sur le produit initial

$$RR = \frac{\text{nombre de moles de produit final}}{\text{nombre de moles de produit initial}}$$

RT = le rendement sur le produit transformé

$$RT = \frac{\text{nombre de moles de produit final}}{\text{nombre de moles de produit transformé}}$$

EXEMPLE 1 - Invention

Dans un ballon de 100 ml, on introduit 4 grammes de N,N-diallyl métatrifluorométhylaniline (m-TFMA) (16,6 mmoles) avec 5 g de chlorure de dichloroacétyle (33,9 mmoles) et on chauffe le mélange à 100 ° C pendant 29 h. Le mélange réactionnel est dilué par 5 ml d'acétate d'éthyle et dosé par chromatographie en phase gazeuse.

| | |
|---|---|
| TT N,N-diallyl m-TFMA | = 73,38 % |
| RT N-dichloroacétyl N-allyl m-TFMA | = 80,13 % |

EXEMPLE 2 - Invention

Dans un ballon de 10 ml, on introduit 1,0195 g de N,N-diallyl m-TFMA (4,34 mmoles) avec 1,231 g de chlorure de dichloroacétyle (8,34 mmoles) avec 0,2264 g de $Pd(DBA)_2$ et 2 ml d'acétonitrile. On maintient le mélange réactionnel à 20 ° C pendant 48 h, on dilue par 5 ml d'acétate d'éthyle puis on dose par chromatographie en phase gazeuse.

| | |
|---|---|
| TT N,N-diallyl m-TFMA | = 63,2 % |
| RT N-dichloroacétyl N-allyl m-TFMA | = 36,6 % |

EXEMPLE 3 - Invention

Dans un ballon de 10 ml, on introduit 0,9940 g de N,N-diallyl m-TFMA (4,12 mmoles) avec 1,2045 g de chlorure de dichloroacétyle (8,16 mmoles), 0,1318 g de $PdCl_2(ØCN)_2$ (0,42 mmoles) et 2 ml d'acétonitrile. On maintient le mélange réactionnel à 20 ° C pendant 48 h puis on dose par chromatographie en phase gazeuse.

| | |
|---|---|
| TT N,N-diallyl m-TFMA | = 39,5 % |
| RT N-dichloroacétyl N-allyl m-TFMA | = 60,3 % |

EXEMPLE 4 - Invention

Dans un ballon de 10 ml, on introduit 2,0171 g de N,N-diallyl m-TFMA (8,37 mmoles) avec 2,44 g de chlorure de dichloroacétyle (16,54 mmoles), 0,4816 g de $PdCl_2$ (0,838 mmoles) et 4 ml d'acétonitrile. On porte le mélange réactionnel à 90°C pendant 3 h 30 puis on dose par chromatographie en phase gazeuse.

| | |
|---|---|
| TT N,N-diallyl m-TFMA | = 100 % |
| RT N-dichloroacétyl N-allyl m-TFMA | ≧ 95 % |

EXEMPLE 5 - Invention

Dans un ballon de 10 ml, on introduit 2,0057 g de N,N-diallyl m-TFMA (8,3 mmoles) avec 2,4563 g de chlorure de dichloroacétyle (16,6 mmoles), 0,0465 g de $PdCl_2$ $(DBA)_2$ (0,08 mmoles) et 4 ml d'acétonitrile. On porte le mélange réactionnel à 80-85°C pendant 7 h 40, puis on dose par chromatographie en phase gazeuse.

| | |
|---|---|
| TT N,N-diallyl m-TFMA | = 100 % |
| RT N-dichloroacétyl N-allyl m-TFMA | ≧ 95 % |

EXEMPLE 6 à 9 - Influence du solvent

Dans un réacteur de 30 ml, on charge

- 0,48 g de N,N-diallyl m-trifluorométhylaniline (2 mM)
- 0,58 g de chlorure de dichloracétyle (4 mM)
- 2 ml de solvant
- 0,2 mM de $Pd(DBA)_2$.

Ce mélange est chauffé 10 h 40 à 75°. Après refroidissement on ajoute 5 ml de soude N. Les produits organiques sont extraits par 5 x 5 ml d'éther isopropylique. La phase organique est filtrée sur clarcel et diluée à 25 ml pour être dosée par étalonnage interne en chromatographie en phase gazeuse.

| N° de l'essai | Solvants | T T NN Di | R T NAND |
|---|---|---|---|
| 6 | $CH_3CN$ | 98,4 | 92,4 |
| 7 | Benzène | 99,9 | 87,2 |
| 8 | Benzonitrile | 96,4 | 100 |
| 9 | Heptane | 99,6 | 85,3 |
| 10 | $H_2O$ | 42,8 | 5,28 |

EXEMPLE 11 - Influence de la durée

On opère comme dans l'exemple 6 avec une durée de 5 heures.

| N° de l'essai | Solvants | TT NN Di | R T NAND |
|---|---|---|---|
| 11 | Heptane | 100 | 66,8 |

EXEMPLES 12 à 16 - Influence du catalyseur, de la température et du temps

On opère comme dans l'exemple 6 en chargeant 0,2 mM de catalyseur.

| N° Essai | Conditions | Solvant | Catalyseur | TT % NN Di | RT % NAND |
|---|---|---|---|---|---|
| Comparatif 1 | 75°C - 7 h | $CH_3CN$ | Sans | 7,1 | 79,3 |
| 12 | 75 °C - 10 h 40 | $CH_3CN$ | $Pd(DBA)_2$ | 98,4 | 92,4 |
| 13 | 75°C - 7 h | $CH_3CN$ | $CuCl_2$ | 50,9 | 81,5 |
| 14 | 70°C - 5 h | $CH_3CN$ | $CuCl_2$ | 36 | 53,3 |
| 15 | 70°C - 5 h | $CH_3CN$ | CuCl 10 % | 35 | 50,2 |
| Comparatif 2 | 40°C - 7 h | $CH_3CN$ | Sans | 0 | 0 |
| 16 | 40°C - 7 h | $CH_3CN$ | $Pd(DBA)_2$ | 48,8 | 53 |

EXEMPLES 17 à 25 - Acétylation de mélange N-allyl et N,N-diallyl et influence de la quantité de catalyseurs

On opère comme dans l'exemple 5 en chargeant initialement

- 0,48 g de N,N-diallyl m-trifluorométhylaniline (2mM)
- 0,4 g de N-allyl m-trifluorométhylaniline (2mM)
- 0,58 g de chlorure de dichloroacétyle (4 mM)

- 2 ml de solvant.

| N° de l'essai | Conditions | Solvant | % en mole de catalyseur | | TT N'allyle % | TT NNDi % | RR NAND % |
|---|---|---|---|---|---|---|---|
| Comparatif 3 | 75°C - 7 h 30 | $CH_3CN$ | Sans | | 100 | 0,5 | 52,1 |
| 17 | 75°C - 7 h 30 | $CH_3CN$ | $Pd(DBA)_2$ | 2,5 | 100 | 100 | 104 |
| 18 | 75°C - 7 h 30 | $CH_3CN$ | $Pd(DBA)_2$ | 1 | 100 | 91,5 | 96,9 |
| 19 | 75°C - 7 h 30 | $CH_3CN$ | $Pd(DBA)_2$ | 0,25 | 100 | 51,4 | 75 % |
| 20 | 75°C - 7 h 30 | $CH_3CN$ | $PdCl_2(ØCN)_2$ | 0,25 | 100 | 27,3 | 66,8 |
| 21 | 75°C - 7 h 30 | $CH_3CN$ | $CuCl_2$ | 10 | 100 | 42 | 70,4 |
| 22 | 75°C - 7 h 30 | $CH_3CN$ | $CuCl_2$ | 2,5 | 100 | 21,1 | 58,4 |
| 23 | 75°C - 7 h 30 | $CH_3CN$ | $CuCl_2$ | 0,25 | 100 | 12,2 | 57,9 |
| 24 | 75°C - 7 h 00 | $CH_3CN$ | CuCl | 10 | 98,2 | 44 | 62,4 |
| 25 | 75°C - 7 h 00 | Heptane | $Pd(OAc)_2$ | 10 | 100 | 100 | 74,6 |

## Revendications

1. Procédé de préparation de N-allyl N-acylaniline caractérisé en ce qu'on met en contact une N,N-diallylaniline ou un mélange de N-mono et N,N-diallylanilines avec un halogénure d'acide carboxylique de préférence en présence d'un catalyseur métallique choisi parmi le palladium et le cuivre avec la condition que lorsque le catalyseur est le palladium, on n'utilise pas les phosphines pour en potentialiser l'action.

2. Procédé selon la revendication 1 caractérisé en ce que les N,N-diallylanilines répondent à la formule (I) suivante

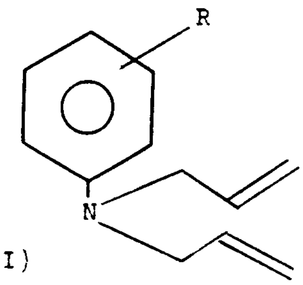

(I)

dans laquelle R représente un groupe halogène, alkyle ayant 1 à 6 atomes de carbone, alkoxy ayant 1 à 6 atomes de carbone, alkylthio ayant 1 à 6 atomes de carbone, halogénoalkyle halogénoalcoxy, halogénoalkylthio dont le groupe alkyle contient 1 à 6 atomes de carbone, nitro, cyano.

3. Procédé selon la revendication 2 caractérisé en ce que R représente le groupe 3-trifluorométhyle.

4. Procédé selon la revendication 1 caractérisé en ce que l'halogénure d'acide est choisi parmi les bromures et les chlorures d'acides carboxyliques contenant 2 à 12 atomes de carbone.

5. Procédé selon la revendication 4 caractérisé en ce que l'halogénure d'acide est le chlorure de dichloracétyle.

6. Procédé selon la revendication 1 caractérisé en ce que le catalyseur métallique est choisi parmi les dichlorures de cuivre et de palladium, le diacétate de palladium, le palladiumdibenzylidèneacétone.

7. Procédé selon la revendication 1 caractérisé en ce que le rapport molaire de l'halogénure d'acide à la N-allylaniline est est supérieur à 1.

8. Procédé selon la revendication 1 caractérisé en ce que le rapport molaire du dérivé métallique à la N-allylaniline est compris entre 0,001 et 0,05.

9. Procédé selon la revendication 1 caractérisé en ce que le température réactionnelle est de préférence comprise entre 20°C et 120°C.

## Claims

1. Process for the preparation of N-allyl-N-acylaniline, characterised in that an N,N-diallylaniline or a mixture of N-mono and N,N-diallylanilines is placed in contact with a carboxylic acid halide, preferably in the presence of a metal catalyst chosen from palladium and copper, with the condition that, when the catalyst is palladium, phosphines are not used to potentiate the action thereof.

2. Process according to Claim 1, characterised in that the N,N-diallylanilines correspond to the following formula (I)

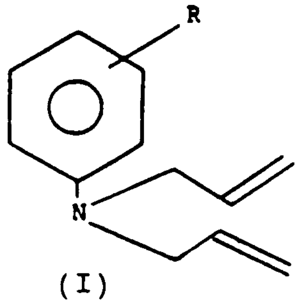

(I)

in which R denotes a halogen, an alkyl containing 1 to 6 carbon atoms, an alkoxy containing 1 to 6 carbon atoms, an alkylthio containing 1 to 6 carbon atoms, a halo-alkyl, a haloalkoxy, a haloalkylthio in which the alkyl group contains 1 to 6 carbon atoms, nitro or cyano group.

**3.** Process according to Claim 2, characterised in that R denotes the 3-trifluoromethyl group.

**4.** Process according to Claim 1, characterised in that the acid halide is chosen from bromides and chlorides of carboxylic acids containing 2 to 12 carbon atoms.

**5.** Process according to Claim 4, characterised in that the acid halide is dichloroacetyl chloride.

**6.** Process according to Claim 1, characterised in that the metal catalyst is chosen from copper and palladium dichlorides, palladium diacetate and dibenzylideneacetonepalladium.

**7.** Process according to Claim 1, characterised in that the molar ratio of the acid halide to the N-allylaniline is greater than 1.

**8.** Process according to Claim 1, characterised in that the molar ratio of the metal derivative to the N-allylaniline is between 0.001 and 0.05.

**9.** Process according to Claim 1, characterised in that the reaction temperature is preferably between 20 ° C and 120 ° C.

## Patentansprüche

**1.** Verfahren zur Darstellung von N-Allyl-N-acylanilin, dadurch gekennzeichnet, daß man ein N,N-Diallylanilin oder eine Mischung von N-Mono- und -N,N-Diallylanilinen mit einem Carbonsäure halogenid in Kontakt bringt, bevorzugt in Anwesenheit eines Metallkatalysators aus Palladium oder Kupfer, unter der Bedingung, daß falls der Katalysator Palladium ist, man keine Phosphine benutzt, um die Wirkung zu steigern.

**2.** Verfahren nach Anspruch 1, dadurch gekennzeichnet, daß die N,N-Diallylaniline der folgenden Formel (I) entsprechen

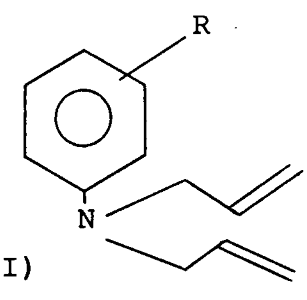

(I)

bei der R eine Halogengruppe repräsentiert, eine Alkylgruppe mit ein bis sechs Kohlenstoffatomen, eine Alkoxygruppe mit ein bis sechs Kohlenstoffatomen, eine Alkylthiogruppe mit eins bis sechs

Kohlenstoffatomen, eine Halogenalkyl-, Halogenalkoxy-, Halogenalkylthiogruppe, deren Alkylrest ein bis sechs Kohlenstoffatome enthält, eine Nitro- oder Cyanogruppe.

**3.** Verfahren nach Anspruch 2, dadurch gekennzeichnet, daß R für eine 3-Trifluormethylgruppe steht.

**4.** Verfahren nach Anspruch 1, dadurch gekennzeichnet, daß als Säurehalogenid Carbonsäurechloride- und -bromide, die 2 bis 12 Kohlenstoffatome enthalten, ausgewählt werden.

**5.** Verfahren nach Anspruch 4, dadurch gekennzeichnet, daß das Säurehalogenid Dichloracetylchlorid ist.

**6.** Verfahren nach Anspruch 1, dadurch gekennzeichnet, daß der metallische Katalysator aus den Kupfer- und Palladiumdichloriden, Palladiumdiacetat und Palladiumdibenzalaceton ausgewählt wird.

**7.** Verfahren nach Anspruch 1, dadurch gekennzeichnet, daß das molare Verhältnis Säurehalogenid zu N-Allylanilin über 1 liegt.

**8.** Verfahren nach Anspruch 1, dadurch gekennzeichnet, daß das molare Verhältnis des Metallderivats zum N-Allylanilin zwischen 0,001 und 0,05 liegt.

**9.** Verfahren nach Anspruch 1, dadurch gekennzeichnet, daß die Reaktionstemperatur bevorzugt zwischen 20 °C und 120 °C liegt.